# EUROPÄISCHE PATENTSCHRIFT

(11) **EP 2 662 102 B1**
(45) Veröffentlichungstag und Bekanntmachung des Hinweises auf die Patenterteilung: **16.03.2016**
(21) Anmeldenummer: 13164849.5
(22) Anmeldetag: 23.04.2013
(51) Int. Cl.: A61M 5/00, B65D 81/113, A61M 5/28, A61M 5/315, A61M 5/31

(54) **Transportträger für Spritzen**
Transport carrier for syringes
Support de transport pour seringues

(30) Priorität: 08.05.2012 CH 6382012
(43) Veröffentlichungstag der Anmeldung: 13.11.2013
(73) Patentinhaber: Fisher Clinical Services GmbH, 4123 Allschwil (CH)
(72) Erfinder: Hunkeler, Guido, 4124 Schönenbuch (CH); Gerstmann, Ulrich, 4123 Allschwil (CH)
(74) Vertreter: Braun, André jr.

(56) Entgegenhaltungen:
- EP-A1- 2 130 561
- WO-A2-01/23017
- CH-A- 360 168
- DE-U1- 8 530 003
- JP-A- 2001 104 475
- US-A- 4 657 138
- US-A- 4 753 345
- US-A1- 2008 255 520

## Beschreibung

Die Erfindung betrifft einen Träger für Spritzen nach dem Oberbegriff des Anspruchs 1.

Bei vorgefüllten Spritzen ist die der Verbindung mit der Injektionsnadel dienende Düse, d.h. meistens der Typ Luer-Lock oder Luer-Slip, für Lagerung und Transport üblicherweise mit einem Stopfen oder dergl. verschlossen, um die Sterilität des Inhalts zu gewährleisten und Leckagen zu verhindern. Es hat sich aber herausgestellt, dass in bestimmten Situationen, z.B. durch Druckunterschiede beim Lufttransport, Verschiebungen des Kolbens vorkommen können, die in mehrfacher Hinsicht nachteilig sind. Dies betrifft vor allem auch die Sterilität des Inhalts, weil der Bereich auf der Rückseite des Kolbens nicht steril ist. Wenn sich also ein Kolben durch Änderungen des Umgebungsdrucks bewegt, kann die Sterilität des Spritzeninhalts gefährdet sein, selbst wenn sich der Kolben nach einer Bewegung wieder am selben Ort befindet.

In WO 01/23017 ist eine Vorrichtung zum Anbringen an einer Spritze beschrieben, mittels der eine relative Bewegung zwischen dem Zylinder der Spritze und der Kolbenstange verhindert wird. Zu diesem Zweck wird um das Betätigungsende der Spritze eine Art Kapsel gelegt, in deren Innerem eine Anzahl Flansche ausgebildet ist, zwischen denen die Flansche des Spritzezylinders und der Kolbenstange gehalten und so axial fixiert werden. Diese Vorrichtung nimmt aber eine Spritze nicht vollständig auf und ermöglicht eine Fixierung der Kolbenstange nur in wenigen diskreten Positionen relativ zum Zylinder.

Ein weiterer aus dem Stand der Technik bekannter Träger zur Aufnahme einer rorgefüllten Spritze ist in JP 2001 104475 A offenbart.

Der Erfindung liegt die Aufgabe zugrunde, unbeabsichtigte Kölbenbewegungen bei vorgefüllten Spritzen während Lagerung und Transport zu verhindern.

Erfindungsgemäss wird dies erreicht durch einen Transportträger mit den kennzeichnenden Merkmalen des Anspruchs 1.

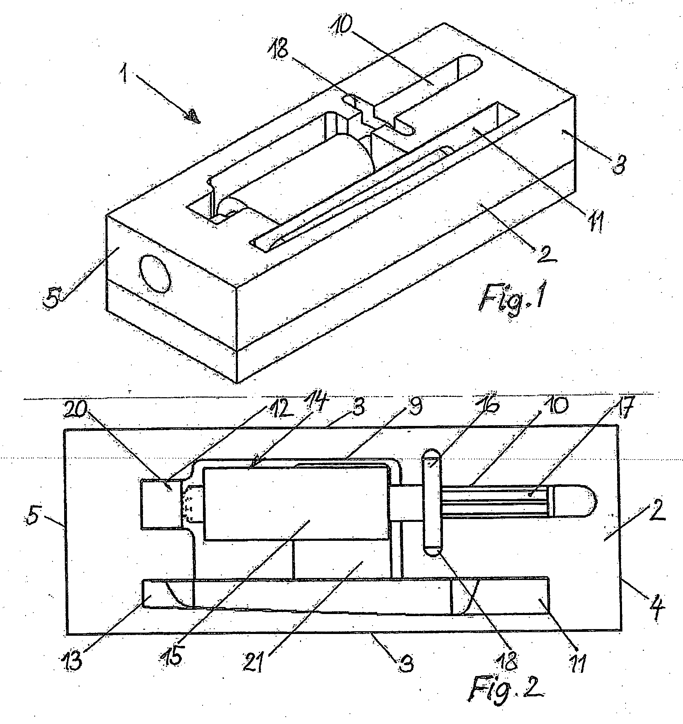

Im Folgenden ist anhand der beiliegenden Zeichnungen ein bevorzugtes Ausführungsbeispiel der Erfindung beschrieben. Es zeigen
- Fig. 1: eine perspektivische Darstellung eines Trägers mit eingelegter Spritze,
- Fig. 2: eine Draufsicht auf den Träger mit eingelegter Spritze

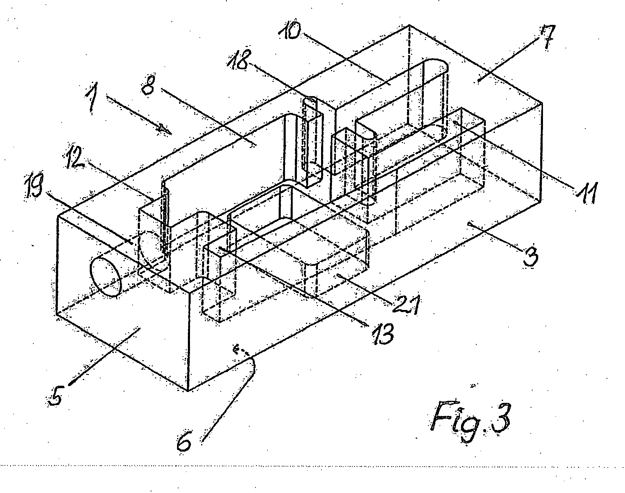

- Fig. 3: eine perspektivische Darstellung des Trägers mit allen in seinem Inneren verlaufenden Konturen

Der in den Figuren gezeigte Träger 1 besitzt die äussere Form eines langgestreckten quaderförmigen Körpers 2 mit zwei einander gegenüber liegenden, geschlossenen Seitenwänden 3, einer ebenfalls geschlossenen, nachfolgend als kolbenseitig bezeichnete Stirnseite 4, einer zweiten, im Folgenden als nadelseitig bezeichneten Stirnseite 5, einer Unterseite 6 und einer Oberseite 7.

Von der Oberseite her erstreckt sich eine Kammer 8 ins Innere des Körpers. Die Kammer umfasst einen mittleren, in der Draufsicht annähernd rechteckigen Bereich 9, von dem aus sich in kolbenseitiger Richtung des Körpers zwei schmale, langgestreckte Ausbuchtungen 10,11 und nadelseitig mit den letzteren dieselben Mittelebenen definierende kürzere Ausbuchtungen 12,13 erstrecken.

Die einander gegenüberliegenden Ausbuchtungen 10,12 auf einer Seite des Körpers dienen mit dem entsprechenden Teil des Mittelbereichs 9 der Aufnahme einer Spritze 14, wobei der Spritzenzylinder 15 im Mittelbereich, der Flansch 16 des Zylinders und die Kolbenstange 17 in der kolbenseitigen Ausbuchtung 10 aufgenommen wird. Für die Aufnahme des Flansches ist ein quer zur Längsrichtung angeordneter Schlitz 18 vorgesehen. Die Kammer ist tief genug, um die Spritze vollständig aufzunehmen.

Von der nadelseitigen Ausbuchtung 12 erstreckt sich eine zylindrische, zur eingelegten Spritze koaxiale Vertiefung 19 in nadelseitiger Richtung. Aus fertigungstechnischen Gründen geht diese Vertiefung bis zur nadelseitigen Stirnseite durch. Diese Vertiefung hat einen dem Verschlussstopfen 20 der Spritze entsprechenden Durchmesser und nimmt den Verschlussstopfen der eingelegten Spritze ganz oder teilweise auf. Selbstverständlich kann auch eine andere Form, z.B. Schlitze oder dergl., zur Aufnahme des Verschlussstopfens dienen.

In der kolbenseitigen Hälfte des Mittelbereichs 9 ist eine Öffnung 21 zur Unterseite des Körpers vorgesehen, die dem Herausnehmen der Spritze aus dem Träger dient. Je nach der für das Herausnehmen der Spritze vorgesehenen Vorkehrung kann ggf. auf die Öffnung 21 verzichtet werden.

Die beiden anderen einander gegenüber liegenden Ausbuchtungen 11,13 dienen zusammen mit dem entsprechenden Teil des Mittelbereichs eine Kammer der Aufnahme einer mit der Spritze zu verwendenden Kanüle.

Der Körper 2 besteht beim vorliegenden Ausführungsbeispiel aus einem hochdichten Schaumstoff, beispielsweise Ethafoam^{R}. Es kann auch ein anderes Material mit ähnlichen Eigenschaften verwendet werden.

Beim Einlegen einer vorgefüllten Spritze in den Träger wird sie zuerst mit dem Verschlussstopfen in die koaxiale Vertiefung eingeführt und dann mit dem Flansch in den Querschlitz 18 eingeschoben. Dadurch ist der Spritzenzylinder in axialer Richtung fixiert. Beim Einschieben des Flansches in den Querschlitz wird gleichzeitig der Kolben in die entsprechende Ausbuchtung eingedrückt. Dabei wird auch die am Ende der Kolbenstange vorhandene flanschförmige Verbreiterung zwischen die Wände der Ausbuchtung gedrückt und in axialer Richtung fixiert. Die Ausbuchtung ist zu diesem Zweck enger als die Breite des Endes der Kolbenstange.

Dadurch dass die flanschförmige Verbreiterung der Kolbenstange zwischen den Seitenwänden der Ausbuchtung gehalten wird, ohne dass dafür eine definierte axiale Position vorgesehen ist, können in demselben Träger Spritzen mit unterschiedlichen Füllständen und entsprechend unterschiedlichen Kolbenpositionen gehalten werden.

Das Herausnehmen der Spritze aus dem Träger erfolgt, indem sie durch die unterhalb befindliche Öffnung nach oben gedrückt wird. Da der Stopfen in der Vertiefung gehalten wird, kann die Nadelseite der Spritze nicht quer zu ihrer Achse bewegt werden, sondern es müssen zuerst das flanschförmige Ende der Kolbenstange und der Flansch des Spritzenzylinders aus der Ausbuchtung heraus geschoben werden, bevor der Spritzenzylinder in axialer Richtung aus der Vertiefung entnommen werden kann. Auf diese Weise ist sichergestellt, dass nicht beim Herausnehmen eine unerwünschte Kolbenverschiebung stattfinden kann. Selbstverständlich kann die Reihenfolge der Schritte beim Einlegen und/oder Herausnehmen der Spritze auch anders erfolgen.

Durchgeführte Tests haben gezeigt, dass mit dem erfindungsgemässen Träger und dieser Fixierung einer Spritze eine axiale Bewegung des Kolbens infolge von Druckunterschieden oder anderen unkontrollierten Einflüssen zuverlässig verhindert wird.

Selbstverständlich liegen unter Verwendung des Erfindungsgedankens andere Ausführungsvarianten nahe. So ist es möglich einen Träger vorzusehen, der keine Aufnahme für eine Kanüle aufweist, sondern allein die Spritze aufnimmt. Für das Herausnehmen sind ebenfalls Varianten möglich, beispielsweise ein unter der Spritze liegendes Band zum Hochziehen der Flansch- bzw. Kolbenseite oder eine Verbreiterung der Kammer neben dem Spritzenzylinder anstelle der Öffnung zur Unterseite.

## Patentansprüche

1. Träger zur Aufnahme einer vorgefüllten Spritze (14) mit einem Körper (2) aus einem formstabilen elastischen Material und einer Kammer (8) zum Einlegen der Spritze mit einem Spritzenzylinder (15), einem im Inneren des Spritzenzylinders mittels einer Kolbenstange (17) axial bewegbaren Kolben und einem an dem der Kolbenstange zugewandten Ende des Spritzenzylinders angeordneten Flansch (16), **gekennzeichnet durch** eine den Flansch des Spritzenzylinders in axialer Richtung fixierende Ausnehmung (18) und eine die Kolbenstange der Spritze in einer beliebigen Position aufnehmende langgestreckte Ausbuchtung (10) der Kammer, die enger ist als eine flanschförmige Verbreiterung am Ende der Kolbenstange derart, dass der Kolben axial fixiert ist.

2. Träger nach Anspruch 1, **dadurch gekennzeichnet, dass** in der Kammer ein Bereich (11, 13) zur Aufnahme einer Kanüle vorgesehen ist.

## Claims

1. A carrier for a pre-filled syringe (14) comprising a main structure (2) made of a dimensionally stable elastic material and a chamber (8) having a syringe cylinder (15) for insertion of syringe, a plunger which is axially movable within the syringe via a plunger rod (17) and a flange (16) disposed at the end of the syringe cylinder facing the piston rod, **characterized by** a recess (18) fixing the flange in the axial direction and by an elongated recess (10) which can accommodate the plunger of the syringe in any position, said elongated recess being narrower than a flange-shaped widening at the end of the plunger rod such that the plunger is axially fixed.

2. A carrier according to claim 1, **characterized in that** an area (11, 13) is provided in the chamber to accommodate a cannula.

## Revendications

1. Support destiné à recevoir une seringue préremplie (14) avec un corps (2) constitué d'un matériau élastique indéformable et une chambre (8) pour l'insertion de la seringue avec un cylindre de seringue (15), un piston déplaçable axialement à l'intérieur du cylindre de seringue au moyen d'une tige de piston (17), et une bride (16) agencée à l'extrémité du cylindre de seringue tournée vers la tige de piston, **caractérisé par** un évidement (18) fixant la bride du cylindre de seringue dans la direction axiale, et une échancrure allongée (10) de la chambre recevant la tige de piston dans une position au choix, laquelle est plus étroite qu'un élargissement en forme de bride situé à l'extrémité de la tige de piston, de sorte que le piston est fixé axialement.

2. Support selon la revendication 1, **caractérisé en ce qu'**une région (11, 13) destinée à recevoir une canule est prévue dans la chambre.
